# EUROPEAN PATENT SPECIFICATION

(11) **EP 3 753 577 B1**
(45) Date of publication and mention of the grant of the patent: **05.04.2023**
(21) Application number: 19754331.7
(22) Date of filing: 13.02.2019
(51) Int. Cl.: A61K 45/00, A61K 31/4468, A61K 38/19, A61P 19/00, A61P 43/00, G01N 33/15, G01N 33/50

(54) **CHONDROCYTE PROLIFERATION PROMOTION METHOD**
VERFAHREN ZUR FÖRDERUNG DER CHONDROZYTENPROLIFERATION
PROCÉDÉ DE PROMOTION DE PROLIFÉRATION DE CHONDROCYTES

(30) Priority: 14.02.2018 JP 2018024297
(43) Date of publication of application: 23.12.2020
(73) Proprietor: Tokyo University of Science Foundation, Tokyo 162-8601 (JP)
(72) Inventor: IWAKURA Yoichiro, Tokyo 162-8601 (JP); MURAYAMA Masanori, Tokyo 162-8601 (JP)
(74) Representative: Isarpatent
(86) International application number: PCT/JP2019/004980
(87) International publication number: WO 2019/159925

(56) References cited:
- WO-A1-2012/157479
- WO-A1-2015/046595
- WO-A1-2015/072544
- CHALLA, T. D. et al.: "Effect of adiponectin on ATDC5 proliferation, differentiation and signaling pathways", Molecular and Cellular Endocrinology, vol. 323, 2010, pages 282-291, XP027054425, ISSN: 0303-7207
- MAEDA, T. et al.: "Cartducin, a Paralog of Acrp30/Adiponectin, Is Induced During Chondrogenic Differentiation and Promotes Proliferation of Chondrogenic Precursors and Chondrocytes", Journal of Cellular Physiology, vol. 206, 2006, pages 537-544, XP055635715, ISSN: 1097-4652
- GARITAONANDIA, I. et al.: "Adiponectin identified as a agonist for PAQR3/RKTG using a yeast-based assay system", Jouranl of Receptor and Signal transduction research, vol. 29, no. 1, 2009, pages 67-73, XP009137577, ISSN: 1532-4281

## Description

### TECHNICAL FIELD

The present invention relates to a protein for use in treating cartilage injury, and an *in vitro* chondrocyte proliferation promotion method.

### BACKGROUND ART

Osteoarthritis is a disease which causes degeneration or deformation of bone tissue due to, for example, wear of the articular cartilage and which frequently develops especially in the elderly. Conventionally, for example, a nonsteroidal anti-inflammatory drug has been used for treating osteoarthritis, but this treatment is no more than symptomatic treatment.

Therefore, in recent years, in order to treat or prevent a cartilage disorder such as osteoarthritis, attempts have been made to proliferate chondrocytes themselves. Hitherto, there have been proposed, as ingredients having a chondrocyte proliferation promotion action, for example, particular polysaccharides, particular urea derivatives, and particular peptides (see, for example, Patent Documents 1 to 3). However, none of the ingredients have achieved practical use, and thus there is a continuing need to search for novel active ingredients.
Patent Document 1: Japanese Unexamined Patent Application, Publication No. 2004-002375
Patent Document 2: Japanese Unexamined Patent Application, Publication No. 2005-336174
Patent Document 3: Japanese Unexamined Patent Application, Publication No. 2015-059087

Challa, T. D. et al. (Molecular and Cellular Endocrinology, 2010, vol. 323, pages 282-291) describe that adiponectin increases chondrocytes proliferation.

Maeda, T. et al. (Journal of Cellular Physiology, 2006, vol. 206, pages 537-544) describe that cartducin (CTRP3) promote the proliferation of chondrocytes.

WO-A-2015072544 describes that cartducin is also known as CTRP3 or cartnectin or CORS-26.

Garitaonandia, I. et al. (Journal of Receptor and Signal transduction research, 2009, vol. 29, no. 1, pages 67-73) describe that adiponectin was concluded not to be an agonist of PAQR4 using a screening method.

WO-A-2012157479 describes that CTRP6 for the prevention or treatment of autoimmune disease such as osteoarthritis, while mentioning that the family member CTRP3 promotes the differentiation and growth of cartilage cells.

WO-A-2015046595 describes that AdipoRon as agonist of PAQR1 and PAQR2.

### DISCLOSURE OF THE INVENTION

### Problems to be Solved by the Invention

An object of the present invention is to provide a novel protein for use in treating cartilage injury according to claim 1, and an *in vitro* chondrocyte proliferation promotion method according to claim 2.

### Means for Solving the Problems

Specific means for solving the above-mentioned problems include the following embodiments.
<1> A protein for use in treating cartilage injury, the protein being any of the following (a) to (c):
   (a) a protein consisting of an amino acid sequence set forth in SEQ ID No: 3;
   (b) a protein consisting of a gClq domain in the amino acid sequence set forth in SEQ ID No: 3;
   (c) a protein having a sequence identity of 80% or more to the gClq domain in the amino acid sequence set forth in SEQ ID No: 3 and acting as an agonist for adiponectin receptor 1 (PAQR1) and adiponectin receptor 4 (PAQR4).
<2> An *in vitro* chondrocyte proliferation promotion method, the method comprising:
   culturing a cartilage tissue or chondrocyte in a medium containing any of the following proteins (a) to (c):
   (a) a protein consisting of an amino acid sequence set forth in SEQ ID No: 3;
   (b) a protein consisting of a gClq domain in the amino acid sequence set forth in SEQ ID No: 3;
   (c) a protein having a sequence identity of 80% or more to the gClq domain in the amino acid sequence set forth in SEQ ID No: 3 and acting as an agonist for adiponectin receptor 1 (PAQR1) and adiponectin receptor 4 (PAQR4).

Meanwhile, adiponectin receptor 1 (AdipoR1) and adiponectin receptor 2 (AdipoR2) are receptors classified as Class I of PAQR (progestin and AdipoQ receptors) family, and also referred to as PAQR1 and PAQR2, respectively. Furthermore, PAQR3, which is also classified as Class I of PAQR family, is also referred to as adiponectin receptor 3 (AdipoR3) since it binds to adiponectin similarly to the AdipoR1 and the AdipoR2. Therefore, PAQR4, which is classified as Class I of PAQR family similarly to these three receptors, is herein defined as adiponectin receptor 4 (AdipoR4).

### Effects of the Invention

According to the present invention, a novel protein for use in treating cartilage injury according to claim 1, and an *in vitro* chondrocyte proliferation promotion method according to claim 2 are provided.

### BRIEF DESCRIPTION OF THE DRAWINGS

FIG. 1A is a graph illustrating incidence rates of osteoarthritis in C1qtnf6^{-/-} mice and wild-type mice.
FIG. 1B is a figure illustrating results of histopathology of tissue sections of ankle joints.
FIG. 2A is a graph illustrating chondrogenesis in primary chondrocytes of neonates of C1qtnf6^{-/-} mice and wild-type mice.
FIG. 2B is a graph illustrating chondrogenesis when primary chondrocytes of C1qtnf6^{-/-} mice were cultured in the presence of various concentrations of recombinant human CTRP6.
FIG. 3A is a graph illustrating the number of cells when ATDC5 cells were cultured in the presence of various concentrations of recombinant human CTRP6.
FIG. 3B is a figure illustrating results of western blotting of MAPKs and phosphorylated MAPKs when ATDC5 cells were cultured in the presence of recombinant human CTRP6.
FIG. 3C is a graph illustrating the number of cells when ATDC5 cells were cultured in the presence or absence of recombinant human CTRP6 and in the presence of MAPK inhibitors.
FIG. 4A is a graph illustrating the number of cells when ATDC5 cells into which AdipoR1 siRNA, AdipoR2 siRNA, or control siRNA-1 had been introduced were cultured in the presence or absence of recombinant human CTRP6.
FIG. 4B is a graph illustrating the number of cells when ATDC5 cells into which AdipoR3 siRNA, AdipoR4 siRNA, or control siRNA-2 had been introduced were cultured in the presence or absence of recombinant human CTRP6.
FIG. 4C is a figure illustrating results of western blotting of ERK and p-ERK when ATDC5 cells into which AdipoR1 siRNA, AdipoR2 siRNA, or control siRNA-1 had been introduced were cultured in the presence of recombinant human CTRP6.
FIG. 4D is a figure illustrating results of western blotting of ERK and p-ERK when ATDC5 cells into which AdipoR3 siRNA, AdipoR4 siRNA, or control siRNA-2 had been introduced were cultured in the presence of recombinant human CTRP6.
FIG. 5A is a graph illustrating the number of cells when ATDC5 cells were cultured in the presence or absence of recombinant human CTRP6 and in the presence or absence of the AdipoR1 blocker.
FIG. 5B is a graph illustrating the number of cells when ATDC5 cells were cultured in the presence or absence of adiponectin and in the presence or absence of the AdipoR1 blocker.
FIG. 5C is a graph illustrating the number of cells when ATDC5 cells were cultured in the presence or absence of AdipoRon and in the presence or absence of the AdipoR1 blocker.
FIG. 5D is a graph illustrating the number of cells when ATDC5 cells were cultured in the presence or absence of recombinant human CTRP3 and in the presence or absence of the AdipoR1 blocker.
FIG. 6A is a graph illustrating the number of cells when ATDC5 cells into which AdipoR1 siRNA, AdipoR2 siRNA, or control siRNA-1 had been introduced were cultured in the presence or absence of recombinant human CTRP3.
FIG. 6B is a graph illustrating the number of cells when ATDC5 cells into which AdipoR3 siRNA, AdipoR4 siRNA, or control siRNA-2 had been introduced were cultured in the presence or absence of recombinant human CTRP3.

### PREFERRED MODE FOR CARRYING OUT THE INVENTION

Embodiments of the present invention will now be described. However, the present invention is not limited to the following embodiments.

### <Chondrocyte proliferation promoter>

Described and not claimed *per se* is a chondrocyte proliferation promoter contains, as an active ingredient, an agonist (hereinafter referred to as "particular AdipoR agonist") for at least one receptor selected from adiponectin receptor 1 (AdipoR1), adiponectin receptor 2 (AdipoR2), adiponectin receptor 3 (AdipoR3), and adiponectin receptor 4 (AdipoR4) .

The present inventors conducted extensive studies, and have confirmed that the particular AdipoR agonist has a proliferation promotion action on a chondrocyte. According to the present embodiment, a novel protein for use in treating cartilage injury according to claim 1 is provided. Furthermore, according to the present embodiment, an *in vitro* chondrocyte proliferation promotion method according to claim 2is also provided.

Generally, particular AdipoR agonist is a material capable of activating at least one receptor selected from AdipoR1, AdipoR2, AdipoR3, and AdipoR4. For example, the particular AdipoR agonist may be a selective agonist for any one receptor selected from AdipoR1, AdipoR2, AdipoR3, and AdipoR4 or an agonist for any plurality of the receptors. Furthermore, as long as the particular AdipoR agonist is capable of activating at least one receptor selected from AdipoR1, AdipoR2, AdipoR3, and AdipoR4, it may be capable of activating another receptor.

Example of the particular AdipoR agonist includes at least one selected from adiponectin, CTRP3, and CTRP6, wherein only CTRP6, but not CTRP3, is involved in the present invention according to claims 1 and 2.

The adiponectin, the CTRP3, and the CTRP6 are all proteins belonging to CTRP (C1q / TNF-related protein) family. The adiponectin, the CTRP3, and the CTRP6 exhibit a chondrocyte proliferation promotion action via activation of AdipoR1, AdipoR2, and AdipoR1 and AdipoR4, respectively, wherein only CTRP6, but not CTRP3, is involved in the present invention according to claims 1 and 2.

Only CTRP6, but not CTRP3, is involved in the present invention according to claims 1 and 2. Origins of the adiponectin, the CTRP3, and the CTRP6 are not particularly limited, and may be human or animals such as mouse, but are preferably human. For amino acid sequences of human adiponectin, human CTRP3, and human CTRP6, one may refer to information registered in the GenBank database at the National Center for Biotechnology Information (NCBI). For example, amino acid sequences of human adiponectin, human CTRP3, and human CTRP6 are deposited in the GenBank database, for example, under Accession Nos. AK291525, AAI12926, and CAK54433, respectively. Typical amino acid sequences are as follows. Underlined portions in the amino acid sequences represent globular domains (gC1q domains).
(Amino acid sequence of human adiponectin (SEQ ID NO: 1)) 
MLLLGAVLLLLALPGHDQETTTQGPGVLLPLPKGACTGWMAGIPGHPGHNGAPGRDGRDGTP
GEKGEKGDPGLIGPKGDIGETGVPGAEGPRGFPGIQGRKGEPGEGAYVYRSAFSVGLETYVT
IPNMPIRFTKIFYNQQNHYDGSTGKFHCNIPGLYYFAYHITVYMKDVKVSLFKKDKAMLFTY
DQYQENNVDQASGSVLLHLEVGDQVWLQVYGEGERNGLYADNDNDSTFTGFLLYHDTN
(Amino acid sequence of human CTRP3 (SEQ ID NO: 2)) 
MLWRQLIYWQLLALFFLPFCLCQDEYMESPQTGGLPPDCSKCCHGDYSFRGYQGPPGPPGPP

GIPGNHGNNGNNGATGHEGAKGEKGDKGDLGPRGERGQHGPKGEKGYPGIPPELQIAFMASL

ATHFSNQNSGIIFSSVETNIGNFFDVMTGRFGAPVSGVYFFTFSMMKHEDVEEVYVYLMHNG

NTVFSMYSYEMKGKSDTSSNHAVLKLAKGDEVWLRMGNGALHGDHQRFSTFAGFLLFETK
(Amino acid sequence of human CTRP6 (SEQ ID NO: 3))

MQWLRVRESPGEATGHRVTMGTAALGPVWAALLLFLLMCEIPMVELTFDRAVASGCQRCCDS

EDPLDPAHVSSASSSGRPHALPEIRPYINITILKGDKGDPGPMGLPGYMGREGPQGEPGPQG

SKGDKGEMGSPGAPCQKRFFAFSVGRKTALHSGEDFQTLLFERVFVNLDGCFDMATGQFAAP

LRGIYFFSLNVHSWNYKETYVHIMHNQKEAVILYAQPSERSIMQSQSVMLDLAYGDRVWVRL

FKRQRENAIYSNDFDTYITFSGHLIKAEDD

Only CTRP6, but not CTRP3, is involved in the present invention according to claims 1 and 2. The adiponectin, the CTRP3, and the CTRP6 may be commercial products or may be produced by a gene recombination method or a chemical synthesis method. When the adiponectin, the CTRP3, or the CTRP6 are produced by the gene recombination method, for example, a gene encoding adiponectin, CTRP3, or CTRP6 may be introduced into a microorganism such as E. coli or yeast, a plant cell, an insect cell, or an animal cell using an expression vector, to thereby express a protein therefrom. When the human adiponectin, the human CTRP3, or the human CTRP6 are produced, a mammalian cell is preferably used from the viewpoints of holding of conformation and post-translational modification. When the adiponectin, the CTRP3, or the CTRP6 are produced by the chemical synthesis method, a liquid phase method, a solid phase method, a Boc method, an Fmoc method, and the like may be used singly or in combination.

Generally adiponectin may be a mutated adiponectin comprising an amino acid sequence in which one or several amino acid residues are substituted for, deleted from, or added to an amino acid sequence of wild-type adiponectin as long as it is capable of activating at least one receptor selected from AdipoR1, AdipoR2, AdipoR3, and AdipoR4. Similarly, described and not claimed *per se* is that the CTRP3 and the CTRP6 may be a mutated CTRP3 and a mutated CTRP6 comprising amino acid sequences in which one or several amino acid residues are substituted for, deleted from, or added to amino acid sequences of wild-type CTRP3 and wild-type CTRP6 as long as they are capable of activating at least one receptor selected from AdipoR1, AdipoR2, AdipoR3, and AdipoR4.

Described and not claimed per se is that sequence identity between each mutated amino acid sequence and each wild-type amino acid sequence may be, for example, 80% or more, 85% or more, 90% or more, 93% or more, or 95% or more. As used herein, the phrase "sequence identity" refers to consistency between sequences when two amino acid sequences are aligned, and may be calculated using, for example, BLAST program (https://www.ncbi.nlm.nih.gov/Blast/cgi) .

Meanwhile, for proteins belonging to CTRP family such as the adiponectin, only a globular domain (gC1q domain) thereof may be present in vivo, therefore, the globular domain is believed to be a functional site thereof (Trends. Immunol., 25 (10): 551-61 (2004)). Consequently, in the present invention, the particular AdipoR agonist may be a peptide constituting the globular domain of the adiponectin CTRP6.

Furthermore, in the present invention, the particular AdipoR agonist may be a peptide constituting a mutated globular domain comprising an amino acid sequence in which one or several amino acid residues are substituted for, deleted from, or added to an amino acid sequence of the globular domain of the adiponectin, CTRP6 according to claim 1. In this case, sequence identity between each mutated amino acid sequence and each wild-type amino acid sequence is 80% or more, preferably 85% or more, 90% or more, 93% or more, or 95% or more.

According to claim 1, particular AdipoR agonists are those selected from the following (a) to (c):
(a) a protein consisting of an amino acid sequence set forth in SEQ ID No: 3;
(b) a protein consisting of a gClq domain in the amino acid sequence set forth in SEQ ID No: 3;
(c) a protein having a sequence identity of 80% or more to the gClq domain in the amino acid sequence set forth in SEQ ID No: 3 and acting as an agonist for AdipoR1 and AdipoR4.

Sequence identity in (c) above may be 85% or more, 90% or more, 93% or more, or 95% or more.

Described and not claimed *per se* is particular AdipoR agonist includes a compound represented by Formula (1) below. The compound represented by Formula (1) below is known to be an agonist for AdipoR1 and AdipoR2 (see, WO2015/046595).

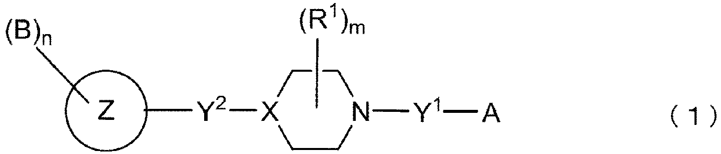

In which
A represents a substituted or unsubstituted aryl group, a substituted or unsubstituted heteroaryl group, a substituted or unsubstituted aryloxy group, a C₄₋₈ tert-alkyl group, or - NH₂;
Y¹ represents -(CHR²)ₐ- in which R² represents a hydrogen atom or a C₁₋₇ alkyl group and a represents an integer of 0 to 2, or -CO-;
X represents CH or N;
R¹ represents a C₁₋₇ alkyl group;
m represents an integer of 0 to 4, when m is 2 or more, an m number of R¹s may be the same as or different from each other;
   i) when X is CH,
      Y² represents *-O-CH₂-CONH-, -O-, *-CONH-, or a group represented by Formula (2) below: 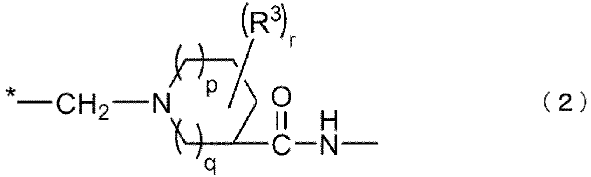
      in which R³ represents a C₁₋₇ alkyl group, p and q each independently represents an integer of 0 to 2, r denotes an integer of 0 to 4, when r is 2, two R³s may be the same as or different from each other, and * denotes an attachment point to Z;
   ii) when X is N,
      Y² represents *-CONH-(CH₂)_{b}-CO-, *-NHCO-Ar¹-CH₂-, *-NHCO-(CH₂)_{b}-, or -CO- in which Ar¹ represents a substituted or unsubstituted arylene group, b represents an integer of 1 to 3, and * represents an attachment point to Z;
      Z represents a cyclic group selected from an aryl group, a heteroaryl group, and a C₃₋₇ cycloalkyl group;
      B is a group which may be substituted on the cyclic group represented by Z, and represents -CO-R⁴, -O-R⁴ in which R⁴ represents a C₁₋₇ alkyl group, a substituted or unsubstituted phenyl group, or a substituted or unsubstituted pyridyl group; -CONR⁵R⁶ in which R⁵ represents a hydrogen atom, a C₃₋₇ cycloalkyl group, a C₁₋₄ alkoxy-C₁₋₄ alkyl group, a norbornenyl-C₁₋₄ alkyl group, or an Ar²-C₁₋₄ alkyl group in which Ar² represents a substituted or unsubstituted aryl group or a substituted or unsubstituted heteroaryl group, and R⁶ represents a hydrogen atom, a C₁₋₇ alkyl group, a C₂₋₄ alkenyl group, or a C₂₋₄ alkynyl group; a C₁₋₇ alkyl group, a C₃₋₇ cycloalkyl group, a halo-C₁₋₇ alkyl group, a phenyl group, a halogen atom, -NO₂, or groups represented by the following formulas: 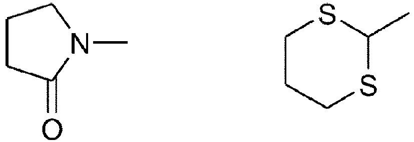
      n represents an integer of 0 to 3, when n is 2 or more, an n number of Bs may be the same as or different from each other.

In Formula (1) above, the "C₁₋₇ alkyl group" includes a methyl group, an ethyl group, an n-propyl group, an isopropyl group, an n-butyl group, an isobutyl group, an s-butyl group, a t-butyl group, an n-pentyl group, an n-hexyl group, an n-heptyl group, and the like, and is preferably a C₁₋₄ alkyl group.

The "aryl group" includes a C₆₋₁₄ aryl group such as a phenyl group, a naphthyl group, an indenyl group, an anthryl group, and the like, is preferably a C₆₋₁₀ aryl group, and is more preferably a phenyl group.

The "heteroaryl group" includes a 5- to 14-membered heteroaryl group such as a furyl group, a thienyl group, an oxazolyl group, a pyridyl group, a pyrimidinyl group, a pyrazolyl group, a benzofuranyl group, a benzoxadiazolyl group, and the like, is preferably a 5- or 6-membered heteroaryl group, and is more preferably a furyl group, a pyridyl group, and a benzofuranyl group.

Aryl in the "aryloxy group" may be the same as those described for the aryl group and is preferably a phenoxy group.

The "arylene group" includes a group in which one hydrogen atom attached to an aromatic ring in the aryl group is removed, and is preferably a phenylene group and a naphthylene group.

A group which may be substituted on the aryl group, the heteroaryl group, the aryloxy group, and the arylene group includes a C₁₋₄ alkyl group (a methyl group, an ethyl group, an n-propyl group, an n-butyl group, an isobutyl group, an s-butyl group, a t-butyl, etc.), a halo-C₁₋₄ alkyl group (a fluoromethyl group, a difluoromethyl group, a trifluoromethyl group, a chloromethyl group, a dichloromethyl group, a pentachloroethyl group, etc.), a halogen atom (a fluorine atom, a chlorine atom, a bromine atom, an iodine atom, etc.), a C₁₋₄ alkoxy group (a methoxy group, an ethoxy group, an n-propoxy group, an isopropoxy group, an n-butoxy group, an isobutoxy group, an s-butoxy group, a t-butoxy group, etc.), a hydroxy group, and the like.

The "C₃₋₇ cycloalkyl group" includes a cyclopropyl group, a cyclobutyl group, a cyclopentyl group, cyclohexyl group, and the like.

The "C₂₋₄ alkenyl group" includes a vinyl group, a propenyl group, and the like.

The "C₂₋₄ alkynyl group" includes an ethynyl group, a propynyl group, a butynyl group, and the like and is preferably a 2-propynyl group and a 2-butynyl group.

The "C₄₋₈ tert-alkyl group" includes a t-butyl group, a t-pentyl group, a t-hexyl group, and the like and is preferably a t-butyl group.

The aryl group represented by A is preferably a phenyl group. Furthermore, the heteroaryl group represented by A is preferably a furyl group, a thienyl group, pyridyl group, a benzofuranyl group, and a benzoxadiazolyl group. Furthermore, the aryloxy group represented by A is preferably a phenoxy group. A group which may be substituted on the aryl group, the heteroaryl group, and the aryloxy group includes a C₁₋₄ alkyl group (a methyl group, an ethyl group, an n-propyl group, an n-butyl group, an isobutyl group, an s-butyl group, a t-butyl, etc.), a halo-C₁₋₄ alkyl group (a fluoromethyl group, a difluoromethyl group, a trifluoromethyl group, a chloromethyl group, a dichloromethyl group, a pentachloroethyl group, etc.), a halogen atom (a fluorine atom, a chlorine atom, a bromine atom, an iodine atom, etc.), a C₁₋₄ alkoxy group (a methoxy group, an ethoxy group, an n-propoxy group, an isopropoxy group, an n-butoxy group, an isobutoxy group, an s-butoxy group, a t-butoxy group, etc.), a hydroxy group, and the like. The substituted aryl group, the substituted heteroaryl group, or the substituted aryloxy group includes an aryl group, heteroaryl group, or aryloxy group which is mono- to tri-substituted with the above-described substituents. A is more preferably a phenyl group or a phenyl group which is mono- to tri-substituted with the above-described substituents.

The -(CHR²)ₐ- represented by Y¹ is preferably those in which R² is a hydrogen atom or a C₁₋₃ alkyl group (preferably a methyl group and an ethyl group) and a is 1, and is more preferably -CH₂- or -CH(CH₃)-.

The C₁₋₇ alkyl group represented by R¹ is preferably a C₁₋₄ alkyl group (a methyl group, an ethyl group, an n-propyl group, an isopropyl group, an n-butyl group, an isobutyl group, an s-butyl group, a t-butyl group, etc.) and is more preferably a methyl group and an ethyl group. Furthermore, m is preferably 0 or 1.

When X is CH, Y² is *-O-CH₂-CONH-, -O-, *-CONH-, or a group represented by Formula (2) above, and is preferably *-O-CH₂-CONH-.

Furthermore, in the group represented by Formula (2) above, it is preferable that both p and q be 0 or one of p and q be 0 and the other be 1, and the C₁₋₇ alkyl group represented by R³ is preferably a C₁₋₄ alkyl group (a methyl group, an ethyl group, an n-propyl group, etc.) and r is preferably 0 or 1. When r is 2, two R³s may be the same as or different from each other.

The group represented by Formula (2) above includes the following groups.

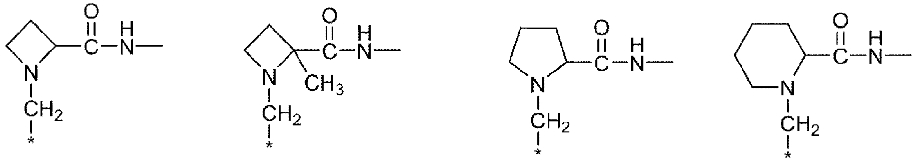

When X is N, Y² is *-CONH-(CH₂)_{b}-CO-, *-NHCO-Ar¹-CH₂-, *-NHCO-(CH₂)_{b}-, or -CO-, is preferably *-CONH-(CH₂)_{b}-CO- and *-NHCO-Ar¹-CH₂-, and is more preferably *-CONH-(CH₂)_{b}-CO-.
b is preferably 2.

The cyclic group represented by Z is an aryl group, a heteroaryl group, or a C₃₋₇ cycloalkyl group, is preferably an aryl group, is more preferably a phenyl group and a naphthyl group, and is further preferably a phenyl group. The "C₃₋₇ cycloalkyl group" is preferably a cyclopropyl group and a cyclobutyl group.

B is a group which may be substituted on the cyclic group represented by Z, and when n is 2 or more, an n number of Bs may be the same as or different from each other. n is preferably an integer of 0 to 2.

In -CO-R⁴ or -O-R⁴ represented by B, R⁴ is preferably a C₁₋₄ alkyl group (a methyl group, an ethyl group, an n-propyl group, etc.), a phenyl group, a phenyl group substituted with mono- or di-substituted with a halogen atom or a nitro group, and a pyridyl group.

In -CONR⁵R⁶ represented by B, R⁵ is preferably a hydrogen atom and a C₃₋₇ cycloalkyl group, R⁶ is preferably a hydrogen atom, a C₁₋₇ alkyl group, and a C₂₋₄ alkenyl group, and more preferably both R⁵ and R⁶ are a hydrogen atom. In R⁵, Ar² in the Ar²-C₁₋₄ alkyl group is preferably a phenyl group, a furyl group, a pyrazolyl group, and a pyridyl group, and C₁₋₄ alkyl in the Ar²-C₁₋₄ alkyl group is preferably C₁₋₂ alkyl. In R⁵, the C₁₋₄ alkoxy-C₁₋₄ alkyl group is preferably a methoxyethyl group, an ethoxyethyl group, and an ethoxypropyl group.

The C₁₋₇ alkyl group represented by B is preferably a methyl group, an ethyl group, an n-propyl group, an isopropyl group, an n-butyl group, an isobutyl group, an s-butyl group, and a t-butyl group. The C₃₋₇ cycloalkyl group represented by B is preferably a cyclopropyl group, a cyclobutyl group, and a cyclopentyl group. The halo-C₁₋₇ alkyl group represented by B is preferably a chloromethyl group, a dichloromethyl group, a fluoromethyl group, a difluoromethyl group, and a trifluoromethyl group, and is more preferably a trifluoromethyl group. The halogen atom represented by B is preferably a fluorine atom, a chlorine atom, a bromine atom, and an iodine atom.

B is more preferably a C₁₋₇ alkyl group, a halogen atom, - NO₂, a phenyl group, a halo-C₁₋₇ alkyl group, -CO-R⁴, -O-R⁴, and -CONR⁵R⁶.

Among compounds represented by Formula (1) above, the following compounds (i) to (vii) are preferable:
(i) a compound in which X is CH and Y² is *-O-CH₂-CONH-, or X is N and Y² is *-CONH-(CH₂)_{b}-CO-;
(ii) a compound in which X is CH, Y² is -O-, Z is a phenyl group in which a group B is at the p (para) position, Y¹ is - CH₂- or -CO-, and A is a substituted or unsubstituted aryl group, a substituted or unsubstituted heteroaryl group, a substituted or unsubstituted aryloxy group, or a C₄₋₈ tert-alkyl group;
(iii) a compound in which X is CH, Y² is *-CONH-, Z is m- or p-chlorophenyl, Y¹ is -CH₂-, and A is a phenyl group;
(iv) a compound in which X is N, Y² is *-NHCO-Ar¹-CH₂-, Ar¹ is a phenyl group, Z is a phenyl group, Y¹ is -CH₂-, and A is a phenyl group;
(v) a compound in which X is N, Y² is *-NHCO-(CH₂)₂-, Z is a cyclopropyl group, Y¹ is -CO-, and A is a phenyl group;
(vi) a compound in which X is N, Y² is *-NHCO-(CH₂)₂-, Z is a phenyl group, Y¹ is a single bond, and A is an o-methoxy phenyl group;
(vii) a compound in which X is N, Y² is -CO-, Z is a phenyl group, B is p- (and m-) methoxy group, Y¹ is -CH₂-, and A is a p-CF₃-phenyl group.

Among the above-described compounds (i), suitable is a compound represented by Formula (1a) or (1b) below: 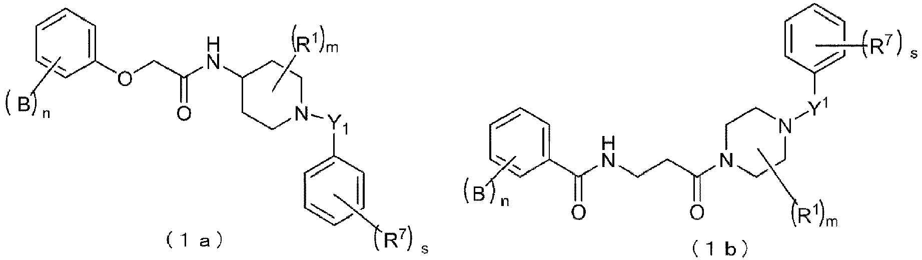 in which Y¹, B, R¹, m, and n have the same meaning as in Formula (1) above, R⁷ represents a C₁₋₄ alkyl group, a halo-C₁₋₄ alkyl group, a halogen atom, a C₁₋₄ alkoxy group, or a hydroxyl group, s represents an integer of 0 to 3, when s is 2 or more, an s number of R⁷s may be the same as or different from each other.

One example of the compound represented by Formula (1a) or (1b) below includes a compound represented by the following formula (also referred to as "AdipoRon").

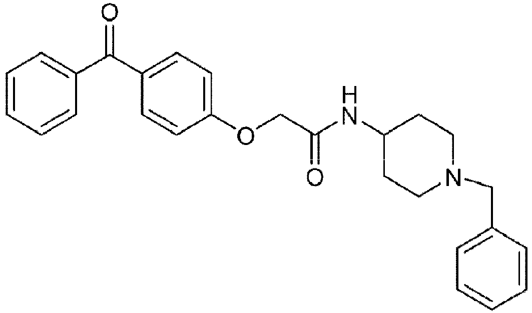

The compound represented by Formula (1) above encompasses all stereoisomers such as geometric isomers (e.g., a cis-isomer or a trans-isomer) and optical isomers (e.g., a d-isomer or an l-isomer), and may be a mixture including the isomers at any proportion.

The compound represented by Formula (1) above may be in the form of an acid addition salt or a base addition salt. Examples of the acid addition salt include a salt with a mineral acid such as hydrochloric acid, sulfuric acid, and the like; a salt with an organic carboxylic acid such as formic acid, acetic acid, citric acid, trichloroacetic acid, trifluoroacetic acid, fumaric acid, maleic acid, and the like; and a salt with a sulfonic acid such as methanesulfonic acid, benzenesulfonic acid, p-toluenesulfonic acid, mesitylenesulfonic acid, naphthalene sulfonic acid, and the like; and the like. Examples of the base addition salt include a salt with an alkali metal such as sodium, potassium, and the like; a salt with an alkali earth metal such as calcium, magnesium, and the like; an ammonium salt; a salt with a nitrogen-containing organic base such as trimethylamine, triethylamine, tributylamine, pyridine, N,N-dimethylaniline, N-methyl-D(-)-glucamine, N-methylpiperidine, N-methylmorpholine, diethylamine, cyclohexylamine, procaine, dibenzylamine, N-benzyl-β-phenethylamine, 1-ephenamine, N,N'-dibenzylethylenediamine, and the like; and the like.

Furthermore, the compound represented by Formula (1) above or salts thereof may be a hydrate or a solvate.

Described and not claimed *per se* is that the chondrocyte proliferation promoter may contain ingredients other than the particular AdipoR agonist, as necessary. Examples of the ingredients other than the particular AdipoR agonist include an excipient, a disintegrant, a binder, a lubricant, a surfactant, a buffer, a solubilizing aid, a stabilizer, a tonicity agent, a suspending agent, an emulsifier, a buffer, a solvent, and the like.

Described and not claimed *per se* is that a dosage form of the chondrocyte proliferation promoter is not particularly limited and may be selected according to applications thereof. For example, when the chondrocyte proliferation promoter is used in medical use, examples of the dosage form of the chondrocyte proliferation promoter include a parenteral agent such as a solution for injection, a lyophilized powder for injection, and the like; an oral agent such as a tablet, a granule, a capsule, a solution, and the like; and the like.

### <Chondrocyte proliferation promotion method>

The chondrocyte proliferation promotion method according to the present embodiment includes bringing the above-mentioned chondrocyte proliferation promoter into contact with a chondrocyte *in vitro.* Described and not claimed *per se* is that the chondrocyte may be a chondrocyte present in vivo.

Described and not claimed *per se* is that examples of the method for bringing the chondrocyte proliferation promoter into contact with the chondrocyte present in vivo include parenteral administration such as injection into the joint, oral administration, and the like. Described and not claimed *per se* is that administration of the chondrocyte proliferation promoter to patients in need of chondrocyte proliferation can promote proliferation of the chondrocyte present in vivo.

Described and not claimed *per se* is that for example, administration of the chondrocyte proliferation promoter to a patient who suffers from a cartilage disorder such as osteoarthritis can treat the cartilage disorder. Furthermore, administration of the chondrocyte proliferation promoter to a patient whose cartilage is injured can ameliorate the cartilage injury and can prevent progression from the cartilage injury to cartilage degeneration and thus to secondary osteoarthritis. Meanwhile, the term "treat" includes disappearance or reduction of symptoms as well as suppression of the degree of symptom progression. Furthermore, the term "prevent" includes prevention of the onset as well as delaying the timing of the onset.

In the present invention, examples of the method for bringing the chondrocyte proliferation promoter into contact with the collected chondrocyte *in vitro* include addition into a medium for culturing a cartilage tissue or a chondrocyte, and the like. Addition of the chondrocyte proliferation promoter into a medium for culturing a collected cartilage tissue or chondrocyte can promote chondrocyte proliferation.

For example, the cartilage disorder or the cartilage injury can be treated or ameliorated by culturing a cartilage tissue or a chondrocyte collected from a patient who suffers from the cartilage disorder such as osteoarthritis or whose cartilage is injured and transplanting or injecting the cultured cartilage tissue or chondrocyte back into the patient.

### <Screening method for chondrocyte proliferation promoters>

Described and not claimed per se is that the screening method for chondrocyte proliferation promoters includes screening candidate materials using, as an indication, activation of at least one receptor selected from AdipoR1, AdipoR2, AdipoR3, and AdipoR4. As described above, the particular AdipoR agonist has the proliferation promotion action on the chondrocyte, therefore, the chondrocyte proliferation promoters can be screened using, as the indication, activation of at least one receptor selected from AdipoR1, AdipoR2, AdipoR3, and AdipoR4. The screening method for chondrocyte proliferation promoters is preferably a method including screening candidate materials using, as the indication, activation of AdipoR4.

Described and not claimed per se is that a method for assessing the activation of at least one receptor selected from AdipoR1, AdipoR2, AdipoR3, and AdipoR4 is not particularly limited and any method can be adopted.

### EXAMPLES

Hereinafter, the present invention will be described in more detail by way of Examples, however, the present invention is not limited to these Examples. Meanwhile, a statistical analysis in Test Example 1 was performed by the chi-square test (**P<0.01), and statistical analyses in Test Examples 2 to 6 were performed by the Student's two-sided t-test (*P<0.05; **P<0.01; ***P<0.001).

### <Test Example 1>

In Test Example 1, KO mice in which C1qtnf6, a gene encoding CTRP6, was knocked out (C1qtnf6^{-/-} mice) were made and verified for the effect of the gene knock out. The C1qtnf6^{-/-}mice were made based on C57BL/6J mice and according to the previous report (Nat. Commun., 6: 8483 (2015)). Wild-type (WT) C57BL/6J mice were purchased from CLEA Japan, Inc.

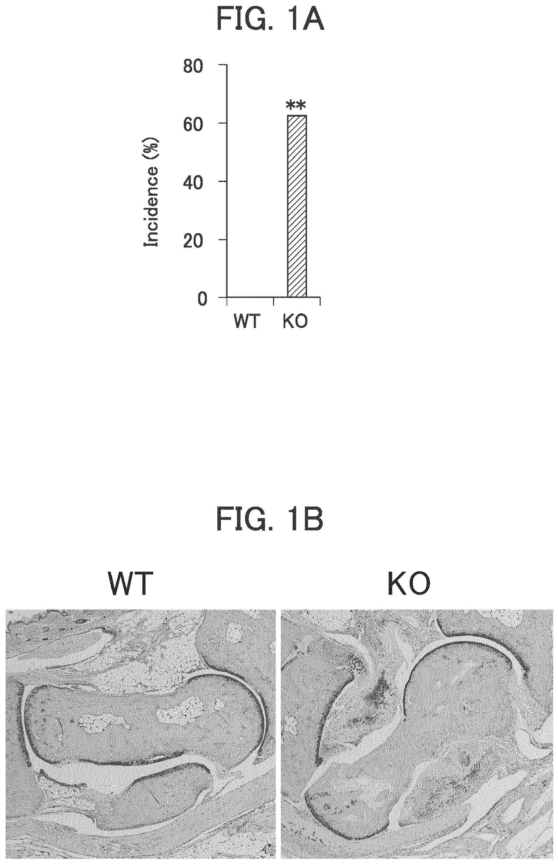

The C1qtnf6^{-/-} mice and the wild-type mice (both 12 month-old) were prepared and visually assessed for the onset of osteoarthritis (n = 8). Furthermore, tissue sections of the ankle joint were made, stained with Safranin O, and subjected to histopathology. Incidence rates of osteoarthritis are presented in FIG. 1A and the results of histopathology are presented in FIG. 1B. It can be seen from the results of FIGs. 1A and 1B that the C1qtnf6^{-/-} mice spontaneously develop osteoarthritis.

### <Test Example 2>

In Test Example 2, a role of CTRP6 in cartilage metabolism was examined.

### (1) Chondrogenesis in C1qtnf6^{-/-} mice and wild-type mice

Costal cartilage tissues from neonates (5 to 6 day-old) of the C1qtnf6^{-/-} mice and the wild-type mice were enzymatically digested with DMEM containing 0.5 mg / ml collagenase D to thereby obtain primary chondrocytes. Then, 2 × 10⁵ cells were suspended into 10 µL of DMEM containing 10% FCS, seeded into a 24-well plate, and cultured at 37°C for 2 hours (both n = 3). Then, DMEM containing 10% FCS was added thereto at a volume of 500 µL per well and culturing was continued for 7 days. Then, the cells were fixed with 10% neutral buffered formalin, washed with 0.1 N HCl, and then stained with a 1% Alcian blue 8GX solution (Sigma-Aldrich) in order to confirm chondrogenesis.

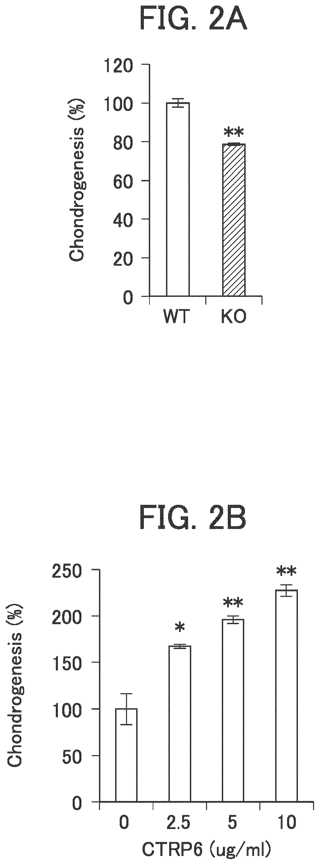

The resultant stained cells were observed with a fluorescent microscope (BIOREVO BZ-9000, KEYENCE CORPORATION) and quantitated for fluorescent intensity using ImageJ software (NIH). The results are presented in FIG. 2A. As illustrated in FIG. 2A, chondrogenesis was more inhibited in the C1qtnf6^{-/-} mice than in the wild-type mice.

### (2) Effect of CTRP6 on chondrogenesis

In the same manner as described above, primary chondrocytes were obtained from the C1qtnf6^{-/-} mice, and then 2 × 10⁵ cells were suspended into 10 µL of DMEM containing 10% FCS, seeded into a 24-well plate, and cultured at 37°C for 2 hours. Then, DMEMs containing 10% FCS and various concentrations (0, 2.5, 5, 10 µg / ml; all n = 3) of recombinant human CTRP6 (Aviscera Bioscience) were added thereto at a volume of 500 µL per well and culturing was continued for 7 days. Then, the cells were fixed with 10% neutral buffered formalin, washed with 0.1 N HCl, and then stained with a 1% Alcian blue 8GX solution (Sigma-Aldrich) in order to confirm chondrogenesis.

The resultant stained cells were observed with a fluorescent microscope (BIOREVO BZ-9000, KEYENCE CORPORATION) and quantitated for fluorescent intensity using ImageJ software (NIH). The results are presented in FIG. 2B. As illustrated in FIG. 2B, in the C1qtnf6^{-/-} mice, the chondrogenesis was promoted in a recombinant human CTRP6 concentration-dependent manner.

### <Test Example 3>

In Test Example 3, a function of CTRP6 was analyzed using a mouse chondrocyte line ATDC5.

### (1) Effect of CTRP6 on cell proliferation of chondrocyte

ATDC5 cells were seeded into a 12-well plate at a cell density of 5 x 10⁴ / well and cultured in the presence of various concentrations (0, 100, 500 ng / ml; all n = 3) of recombinant human CTRP6 (Aviscera Bioscience). As a medium, a HAM's F-12 medium containing 5% FBS and 1% penicillin / streptomycin was used.

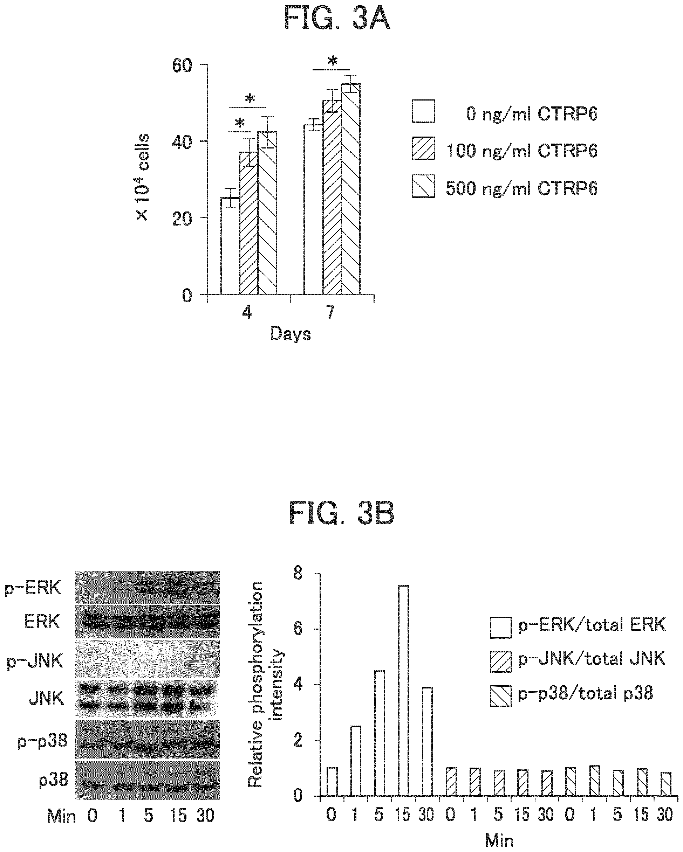

After 4 and 7 day-culture, the number of cells was counted with a hemocytometer. The results are presented in FIG. 3A. As illustrated in FIG. 3A, it was confirmed that addition of the recombinant human CTRP6 facilitated cell proliferation of the ATDC5 cells.

### (2) Effect of CTRP6 on phosphorylation of MAPK

ATDC5 cells were seeded into a 60 mm Petri dish at a cell density of 1 × 10⁶ / well and cultured in a serum-free HAM's F-12 medium for 4 hours. Then, the resultant was washed with PBS and then cultured in a serum-free HAM's F-12 medium containing 10 ng / ml recombinant human CTRP6 (Aviscera Bioscience) for 1 min, 5 min, 15 min, or 30 min. Then, the cells were washed with a phosphatase-inhibiting buffer supplemented with PhosSTOP (Sigma) and then lysed with a lysis buffer (0.1% TritonX-100, 100 mM NaCl, 50 mM Tris-HCl (pH 7.5)).

Then, the resultant cell lysate was subjected to electrophoresis on a 12.5% SDS-PAGE gel and proteins were transferred onto a PVDF membrane. The membrane onto which the proteins had been transferred was blocked by shaking in 5% BSA / TBS at room temperature for 1 hour. After blocking, the membrane was shaken in the presence of a primary antibody (rabbit monoclonal antibody) at room temperature for 1 hour to thereby undergo a primary antibody reaction. As the primary antibody, an anti-ERK1/2 antibody (4695, 1000-fold dilution), an anti-p-ERK1/2 antibody (4370, 1000-fold dilution), an anti-JNK antibody (9258, 1000-fold dilution), an anti-p-JNK antibody (4668, 1000-fold dilution), an anti-p38 antibody (9212, 1000-fold dilution), or an anti-p-p38 antibody (9211, 1000-fold dilution) (all purchased from Cell Signaling) were used. Then, the membrane was washed with TBS containing 0.1% Tween-20 (TBST), and then shaken in the presence of an HRP-labeled anti-rabbit IgG goat polyclonal antibody (Jackson ImmunoResearch, 111-035-144, 2000-fold dilution) at room temperature for 1 hour to thereby undergo a secondary antibody reaction. After washing with TBST three times, ECL Prime western blotting detection system (GE Healthcare) was used to visualize the proteins. Intensity of each band was quantified with ImageJ software (NIH).

The western blotting results are presented in FIG. 3B. As illustrated in FIG. 3B, addition of the recombinant human CTRP6 facilitated phosphorylation of ERK1/2.

### (3) Effect of MAPK inhibitor on cell proliferation of chondrocyte

ATDC5 cells were seeded into a 48-well plate at a cell density of 2 x 10⁴ / well and cultured in the presence or absence of 50 ng / ml recombinant human CTRP6 (Aviscera Bioscience) for 2 days. A HAM's F-12 medium containing 5% FBS and 1% penicillin / streptomycin was used as a medium, and a carrier (DMSO), 1 µM ERK1/2 inhibitor (U0126, Sigma), 1 µM JNK inhibitor (SP600125, Sigma), or 1 µM p38 inhibitor (SB239063, Sigma) was further added thereto (all n = 4).

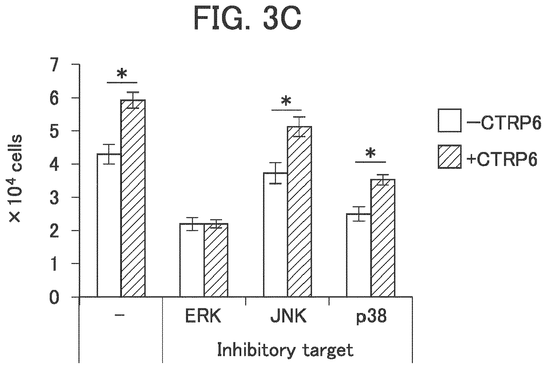

After 2 day-culture, the number of cells was counted with a hemocytometer. The results are presented in FIG. 3C. As illustrated in FIG. 3C, addition of the recombinant human CTRP6 facilitated cell proliferation of the ATDC5 cells, while addition of the ERK1/2 inhibitor inhibited cell proliferation. This suggests that chondrocyte proliferation by CTRP6 is via phosphorylation of ERK1/2.

### <Test Example 4>

In Test Example 4, a receptor of CTRP6 was identified.

### (1) Involvement of adiponectin receptor in cell proliferation of chondrocyte

ATDC5 cells were seeded into a 24-well plate at a cell density of 1 × 10⁶ / well and cultured in a HAM's F-12 medium containing 2.5% FBS for 24 hours. After culturing, 30 pmol of AdipoR1 siRNA, 30 pmol of AdipoR2 siRNA, 30 pmol of control siRNA-1, 6 pmol of AdipoR3 siRNA, 6 pmol of AdipoR4 siRNA, or 6 pmol of control siRNA-2 was introduced (transfected) thereinto and culturing was continued for 48 hours. Lipofectamine RNAiMAX (Invitrogen) was used to introduce the siRNAs.

Meanwhile, the AdipoR3 siRNA, the AdipoR4 siRNA, and the control siRNA-2 were purchased from IDT Teck (TriTECTa RNAi Kit). Base sequences of the AdipoR1 siRNA, the AdipoR2 siRNA, and the control siRNA-1 were as follows:
(Sense strand of AdipoR1 siRNA (SEQ ID NO: 4)) 5'-GAGACUGGCAACAUCUGGACATT-3'
(Antisense strand of AdipoR1 siRNA (SEQ ID NO: 5)) 5'-UGUCCAGAUGUUGCCAGUCUCTT-3'
(Sense strand of AdipoR2 siRNA (SEQ ID NO: 6)) 5'-GCUUAGAGACACCUGUUUGUUTT-3'
(Antisense strand of AdipoR2 siRNA (SEQ ID NO: 7)) 5'-AACAAACAGGUGUCUCUAAGCTT-3'
(Sense strand of control siRNA-1 (SEQ ID NO: 8)) 5'-GUGCGCUGCUGGUGCCAACCCTT-3'
(Antisense strand of control siRNA-1 (SEQ ID NO: 9)) 5'-GGGUUGGCACCAGCAGCGCACTT-3'

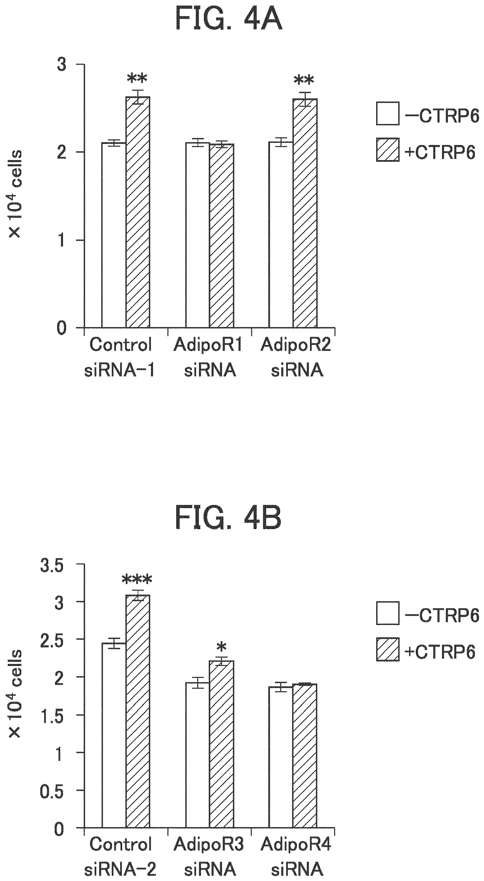

Forty-eight hours after the introduction of the siRNAs, the ATDC5 cells were seeded into a 48-well plate at a cell density of 2 x 10⁴ / well and cultured in the presence or absence of 100 ng / ml recombinant human CTRP6 (Aviscera Bioscience) for 1 day (all n = 4). After that, the number of cells was counted with a hemocytometer. The results are presented in FIGs. 4A and 4B. As illustrated in FIGs. 4A and 4B, in the ATDC5 cells into which the AdipoR1 siRNA or the AdipoR4 siRNA had been introduced, facilitation of cell proliferation was not confirmed even though the recombinant human CTRP6 was added. This suggests that CTRP6 regulates chondrocyte proliferation via AdipoR1 and AdipoR4.

### (2) Involvement of adiponectin receptor in ERK phosphorylation

ATDC5 cells into which the siRNA had been introduced were seeded into a 60 mm Petri dish at a cell density of 1 × 10⁶ / well and cultured in a serum-free HAM's F-12 medium for 4 hours. Then, the cells were washed with PBS and then cultured in a serum-free HAM's F-12 medium containing 10 ng / ml recombinant human CTRP6 (Aviscera Bioscience) for 15 min. Then, the cells were washed with a phosphatase-inhibiting buffer supplemented with PhosSTOP (Sigma) and then lysed with a lysis buffer (0.1% TritonX-100, 100 mM NaCl, 50 mM Tris-HCl (pH 7.5)). Western blotting analysis was performed in the same manner as in Test Example 3 (2).

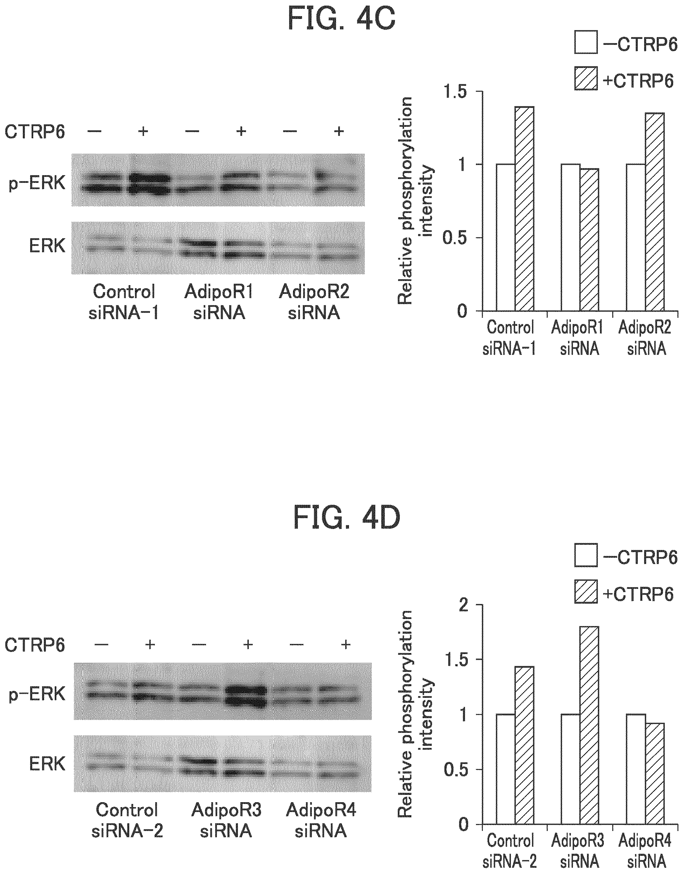

The western blotting results are presented in FIGs. 4C and 4D. As illustrated in FIGs. 4C and 4D, in the ATDC5 cells into which the AdipoR1 siRNA or the AdipoR4 siRNA had been introduced, phosphorylation of ERK1/2 was not facilitated even though the recombinant human CTRP6 was added. This suggests that CTRP6 regulates chondrocyte proliferation via AdipoR1 and AdipoR4.

### <Test Example 5>

In Test Example 5, receptors of adiponectin, AdipoRon, and CTRP3 as well as CTPR 6 were identified.

ATDC5 cells were seeded into a 12-well plate at a cell density of 1 × 10⁴ / well and cultured in the presence or absence of 10 µg / ml AdipoR1 blocker (GeneTex) and in the presence or absence of 100 ng / ml recombinant human CTRP6 (Aviscera Bioscience) for 2 days (all n = 4). As a medium, a HAM's F-12 medium containing 5% FBS and 1% penicillin / streptomycin was used.

Furthermore, 100 ng / ml adiponectin (BioVendor), 100 ng / ml AdipoRon (AdipoGen), or 50 ng / ml recombinant human CTRP3 (Aviscera Bioscience) was used instead of 100 ng / ml recombinant human CTRP6 to culture in the same manner as described above (all n = 4). Meanwhile, when the AdipoRon was used, a culture period was 1 day.

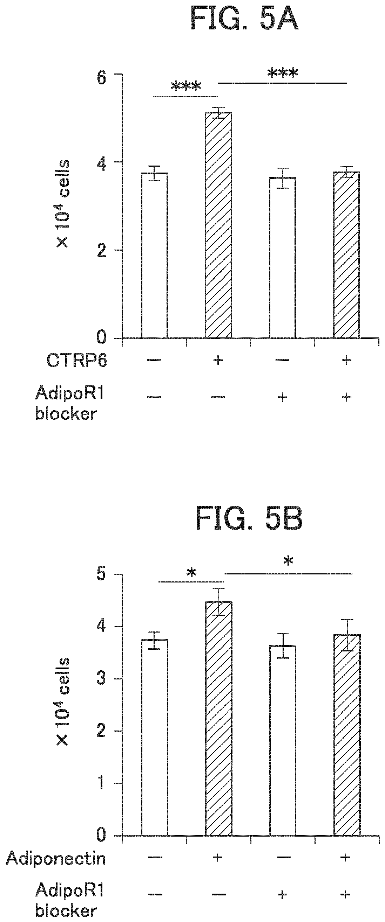

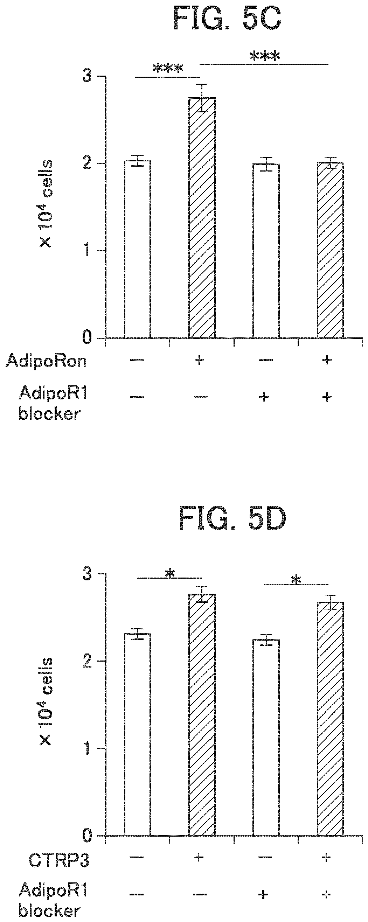

After culturing, the number of cells was counted with a hemocytometer. The results are presented in FIGs. 5A to 5D. As illustrated in FIGs. 5A to 5D, addition of the recombinant human CTRP6, the adiponectin, or the AdipoRon facilitated cell proliferation of the ATDC5 cells, while addition of the AdipoR1 blocker inhibited cell proliferation. Cell proliferation of the ATDC5 cells was also facilitated when the recombinant human CTRP6 was added, but cell proliferation was not inhibited even though the adipoR1 blocker was added. This suggests that the CTRP6, the adiponectin, and the AdipoRon regulate chondrocyte proliferation via AdipoR1.

### <Reference Test Example 6>

In Test Example 6, a receptor of CTRP3 was identified.

AdipoR1 siRNA, AdipoR2 siRNA, control siRNA-1, AdipoR3 siRNA, AdipoR4 siRNA, or control siRNA-2 was introduced (transfected) into ATDC5 cells in the same manner as in Test Example 4 (1).

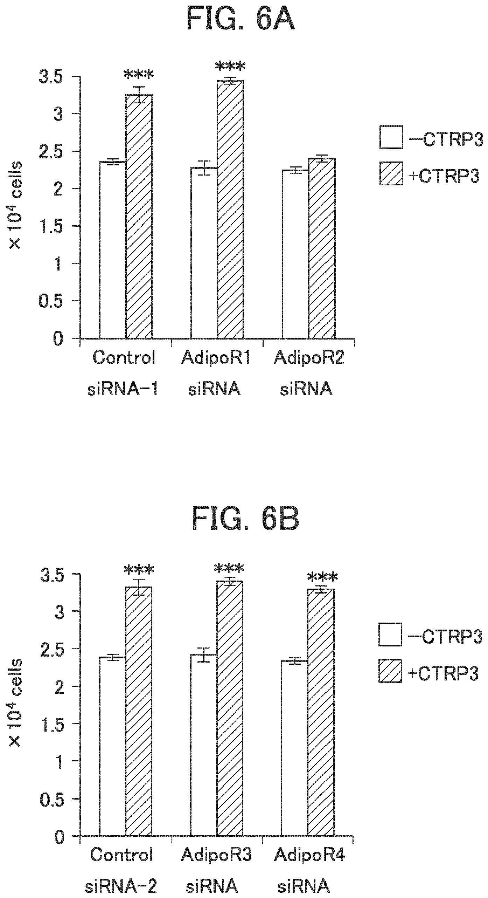

Forty-eight hours after the introduction of the siRNAs, the ATDC5 cells were seeded into a 48-well plate at a cell density of 2 x 10⁴ / well and cultured in the presence or absence of 50 ng / ml recombinant human CTRP3 (Aviscera Bioscience) for 2 days (all n = 4). After that, the number of cells was counted with a hemocytometer. The results are presented in FIGs. 6A and 6B. As illustrated in FIG. 6A and 6B, in the ATDC5 cells into which the AdipoR2 siRNA had been introduced, facilitation of cell proliferation was not confirmed even though the recombinant human CTRP3 was added. This suggests that CTRP3 regulates chondrocyte proliferation via AdipoR2.

## Claims

1. A protein for use in treating cartilage injury, the protein being any of the following (a) to (c):
(a) a protein consisting of an amino acid sequence set forth in SEQ ID No: 3;
(b) a protein consisting of a gClq domain in the amino acid sequence set forth in SEQ ID No: 3;
(c) a protein having a sequence identity of 80% or more to the gClq domain in the amino acid sequence set forth in SEQ ID No: 3 and acting as an agonist for adiponectin receptor 1 (PAQR1) and adiponectin receptor 4 (PAQR4).

2. An *in vitro* chondrocyte proliferation promotion method, the method comprising:
culturing a cartilage tissue or chondrocyte in a medium containing any of the following proteins (a) to (c):
(a) a protein consisting of an amino acid sequence set forth in SEQ ID No: 3;
(b) a protein consisting of a gClq domain in the amino acid sequence set forth in SEQ ID No: 3;
(c) a protein having a sequence identity of 80% or more to the gClq domain in the amino acid sequence set forth in SEQ ID No: 3 and acting as an agonist for adiponectin receptor 1 (PAQR1) and adiponectin receptor 4 (PAQR4).

## Patentansprüche

1. Protein zur Verwendung bei der Behandlung eines Knorpelschadens, wobei das Protein eines der folgenden (a) bis (c) ist:
(a) ein Protein, bestehend aus einer Aminosäuresequenz, dargestellt in SEQ ID Nr. 3;
(b) ein Protein, bestehend aus einer gC1q-Domäne in der in SEQ ID Nr. 3 dargestellten Aminosäuresequenz;
(c) ein Protein, das eine Sequenzidentität von 80 % oder mehr zu der gC1q-Domäne in der in SEQ ID Nr. 3 dargestellten Aminosäuresequenz hat und als Agonist für Adiponectin-Rezeptor 1 (PAQR1) und Adiponectin-Rezeptor 4 (PAQR4) wirkt.

2. Verfahren zur Förderung der Chondrozytenproliferation *in vitro,* wobei das Verfahren umfasst:
Kultivieren eines Knorpelgewebes oder eines Chondrozyten in einem Medium, das eines der folgenden Proteine (a) bis (c) enthält:
(a) ein Protein, bestehend aus einer Aminosäuresequenz, dargestellt in SEQ ID Nr. 3;
(b) ein Protein, bestehend aus einer gC1q-Domäne in der in SEQ ID Nr. 3 dargestellten Aminosäuresequenz;
(c) ein Protein, das eine Sequenzidentität von 80 % oder mehr zu der gC1q-Domäne in der in SEQ ID Nr. 3 dargestellten Aminosäuresequenz hat und als Agonist für Adiponectin-Rezeptor 1 (PAQR1) und Adiponectin-Rezeptor 4 (PAQR4) wirkt.

## Revendications

1. Protéine destinée à être utilisée dans le traitement d'une lésion de cartilage, la protéine étant l'une quelconque des (a) à (c) suivantes :
(a) une protéine constituée d'une séquence d'acides aminés présentée dans SEQ ID n° 3 ;
(b) une protéine constituée d'un domaine gClq dans la séquence d'acides aminés présentée dans SEQ ID n° 3 ;
(c) une protéine ayant une identité de séquence de 80 % ou plus au domaine gClq dans la séquence d'acides aminés présentée dans SEQ ID n° 3 et agissant comme un agoniste pour le récepteur d'adipoectine 1 (PAQR1) et le récepteur d'adiponectine 4 (PAQR4).

2. Procédé de promotion de prolifération de chondrocytes *in vitro,* le procédé comprenant :
la culture d'un tissu cartilagineux ou de chondrocyte dans un milieu contenant l'une quelconque des protéines (a) à (c) suivantes :
(a) une protéine constituée d'une séquence d'acides aminés présentée dans SEQ ID n° 3 ;
(b) une protéine constituée d'un domaine gClq dans la séquence d'acides aminés présentée dans SEQ ID n° 3 ;
(c) une protéine ayant une identité de séquence de 80 % ou plus au domaine gClq dans la séquence d'acides aminés présentée dans SEQ ID n° 3 et agissant comme un agoniste pour le récepteur d'adiponectine 1 (PAQR1) et le récepteur d'adiponectine 4 (PAQR4).
